(19) Europäisches Patentamt
European Patent Office
Office européen des brevets

(11) **EP 2 445 404 B1**

(12) **EUROPÄISCHE PATENTSCHRIFT**

(45) Veröffentlichungstag und Bekanntmachung des Hinweises auf die Patenterteilung:
**10.05.2017 Patentblatt 2017/19**

(21) Anmeldenummer: **10706956.9**

(22) Anmeldetag: **05.03.2010**

(51) Int Cl.:
*A61B 5/103* (2006.01)     *A45D 44/00* (2006.01)

(86) Internationale Anmeldenummer:
**PCT/EP2010/001367**

(87) Internationale Veröffentlichungsnummer:
**WO 2010/149235 (29.12.2010 Gazette 2010/52)**

(54) **VERFAHREN UND VORRICHTUNG ZUR ALTERSBESTIMMUNG UND ALTERSABHÄNGIGEN AUSWAHL VON KOSMETISCHEN PRODUKTEN**

METHOD AND DEVICE FOR DETERMINING AGE, AND AGE-DEPENDENT SELECTION OF COSMETIC PRODUCTS

PROCÉDÉ ET DISPOSITIF POUR LA DÉTERMINATION DE L'ÂGE ET LA SÉLECTION DE PRODUITS COSMÉTIQUES EN FONCTION DE L'ÂGE

(84) Benannte Vertragsstaaten:
**AT BE BG CH CY CZ DE DK EE ES FI FR GB GR HR HU IE IS IT LI LT LU LV MC MK MT NL NO PL PT RO SE SI SK SM TR**

(30) Priorität: **22.06.2009 DE 102009030062**

(43) Veröffentlichungstag der Anmeldung:
**02.05.2012 Patentblatt 2012/18**

(73) Patentinhaber: **Courage + Khazaka electronic GmbH**
**50829 Köln (DE)**

(72) Erfinder:
• **CLEMANN, Sven**
**20253 Hamburg (DE)**
• **JASPERS, Sören**
**22869 Schenefeld (DE)**
• **WOLDE, Maximilian**
**22301 Hamburg (DE)**

• **KHAZAKA, Gabriel**
**50859 Köln (DE)**

(74) Vertreter: **dompatent von Kreisler Selting Werner - Partnerschaft von Patent- und Rechtsanwälten mbB**
**Deichmannhaus am Dom**
**Bahnhofsvorplatz 1**
**50667 Köln (DE)**

(56) Entgegenhaltungen:
**WO-A2-2008/003146     US-A1- 2006 239 547**

• **YURIY VASHPANOV ET AL: "Multispectral images in polarized light for medical applications" MULTISENSOR FUSION AND INTEGRATION FOR INTELLIGENT SYSTEMS, 2008. MFI 2008. IEEE INTERNATIONAL CONFERENCE ON, IEEE, PISCATAWAY, NJ, USA, 20. August 2008 (2008-08-20), Seiten 86-89, XP031346319 ISBN: 978-1-4244-2143-5**

**Beschreibung**

Die Erfindung betrifft ein Verfahren und eine für das Verfahren geeignete Vorrichtung zur Bestimmung des Alters von Konsumenten, mit der Möglichkeit auf das Alter abgestimmte kosmetische Produkte vorzuschlagen. Ein dem Stand der Technik entsprechendes Verfahren zur Bestimmung der Hautalterung ist zum Beispiel in WO 2008/003146 A2 offenbart.

Im Laufe des Lebens verändert sich die Haut eines Menschen. Im Jugendalter stellt sich die Haut in der Regel als straff, glatt, gleichmäßig pigmentiert dar. Mit zunehmendem Alter ändert sich dieses individuell unterschiedlich stark.

Als Hautalterung wird der komplexe biologische Prozess der mit dem Alter einhergehenden Veränderung der Haut bezeichnet. Hierbei ist nicht nur die chronologische Alterung gemeint, sondern auch die intrinsische Alterung, also die genetisch gesteuerte verminderte Reagibilität der Hautzellen. Sie ist nicht beeinflussbar. Im Gegensatz hierzu können die extrinsischen Faktoren (Umweltfaktoren wie UV-Licht, chemische Reagentien, mechanische Belastung) beeinflusst werden. Deshalb unterscheidet man bei der Hautalterung zwischen dem sogenannten "Zeitaltern" und dem "Umweltaltern" (auch "Lichtaltern" genannt). Hautalterung, die meist in Form von Falten sichtbar wird setzt von Mensch zu Mensch unterschiedlich ein, dies ist auch auf die Lebensweise der Person zurückzuführen: Faktoren wie Hitze und Kälte, Stress und falsche Ernährung sowie Alkohol- und Nikotinkonsum können die natürliche Alterung der Haut beschleunigen.

Zeitaltern der Haut kommt jedoch durch eine Erschöpfung der Zellteilungsprozesse und eine Minderversorgung der Zellen zustande. Die Haut bekommt tiefe Falten und Runzeln, ihre trockene Oberfläche neigt zu Einrissen und Pseudonarben, die Oberhaut wird dünner, wodurch die Blutgefäße noch deutlicher hervortreten. Die Trockenheit der alten Haut ist auf eine verminderte Aktivität der Talgdrüsen zurückzuführen: Es wird weniger Fett produziert, die Haut verliert an Elastizität und ist nicht mehr so regenerationsfähig, was insgesamt sogar zu Wundheilungsstörungen führen kann.

Bei manchen Menschen beginnt bereits mit Mitte 20 das Zeitaltern. Im Folgenden findet sich eine Liste der häufigsten Alterserscheinungen verschiedener Altersgruppen. Hierzu ist anzumerken, dass es sich nur um ungefähre Werte handelt, die nicht auf jeden Menschen zutreffen. Die Liste bezieht sich auf Frauen - bei ihnen beginnt die Alterung circa 10 Jahre früher als bei Männern. Dafür schreitet die männliche Hautalterung jedoch schneller voran.

Ab dem Alter von 25 Jahren: Bei manchen Menschen verlangsamt sich bereits in diesem Alter die Zellteilung. Die Oberhaut wird infolgedessen dünner und beginnt, ihre Elastizität zu verlieren.

Ab dem Alter von 30 Jahren: Zwischen den Augen können sich sogenannte Glabellafalten bilden, die durch unsere Mimik hervorgerufen werden. Auch erste Nasolabialfalten (Falten zwischen Nase und Oberlippe) können entstehen. Außerdem bilden sich am Rande der Augen Krähenfüße. Dies sind kleine Fältchen, die zum Auge hin verlaufen und die bei vielen Menschen das erste sichtbare Zeichen der Hautalterung sind.

Ab dem Alter von 40 Jahren: Im Gesicht entstehen mit den sogenannten Knitterfältchen kleine Falten, die - wie der Name schon sagt - den Betroffenen ein zerknittertes Aussehen verleihen. Andere, bereits vorhandene Falten vertiefen sich und die Haut beginnt, stark an Feuchtigkeit zu verlieren. Bei Frauen hinterlässt die Menopause - das Ende der Menstruation - ihre Spuren. Die Produktion des Hormons Östrogen geht zurück, und das hat vielerlei Folgen: Unter anderem wird die Haut dünner und empfindlicher.

Ab dem Alter von 50 Jahren: Die Hautspannung lässt weiter nach. Es entstehen Altersflecken, bei denen sich der Hautfarbstoff Melanin an einer Stelle sammelt und so dunkle Flecken entstehen lässt.

Die zuvor dargestellte grobe Unterteilung in verschiedene Altersstufen der Hautalterung ist jedoch nicht für jeden Menschen Anwendbar. Insbesondere hat die Lebensweise des einzelnen, seine ethnische Herkunft einen entscheidenden Einfluss auf die Ausprägung der oben genannten Merkmale, so dass es zu einer Differenz zwischen wahrem Alter und dem Hautalter kommen kann.

Eine Verbesserung der Hautstruktur und des Erscheinungsbildes, insbesondere die Reduktion von Falten und Pigmentierungsunregelmäßigkeiten, wird durch regelmäßige Anwendung von kosmetischen Pflegeprodukten erreicht. Die Kosmetikindustrie gibt daher zu Ihren Produkten entsprechende Anwendungshinweise ab, die auch einen Hinweis aufweisen, für welche Altersstufe das Produkt geeignet ist.

Liegen wahres Alter des Anwenders und Hautalter auseinander, ist der Verbraucher durch die Anwendungshinweise fehlgeleitet und würde ein Produkt auswählen, das nicht für sein Hautalter geeignet ist.

Aufgabe des erfindungsgemäßen Verfahrens ist es daher, das Hautalter anhand eines Abbildes eines ausgewählten Hautbereiches oder eines Abbildes von mindestens einem Fingernagel zu bestimmen und anzuzeigen oder anhand des bestimmten Alters eine Empfehlung für kosmetische Produkte abzugeben. Verfahren und Vorrichtung der Erfindung sind in den unabhängigen Ansprüchen 1 und 11 aufgeführt. Weitere mögliche Merkmale sind in den abhängigen Ansprüchen aufgeführt.

Die Bestimmung des Hautalters erfolgt insbesondere dadurch, dass ein vorbestimmtes Hautareal, z. B. der Handrücken oder mindestens ein Fingernagel optoelektronisch erfasst wird. Die bei der Erfassung der des Hautabschnittes anfallenden Bilddaten werden in einem Zwischenspeicher abgelegt. Anschließend wird mittels Bildanalyse die Farbverteilung, insbesondere das Verhältnis von dunklen zu hellen Bereichen und/oder der Farbraum, insbesondere das Verhältnis von hellster zu dunkelster Stelle bestimmt. Die Bestimmung erfolgt dabei integrativ, wobei das Ergebnis ebenfalls in einem Zwischenspeicher abgelegt wird.

Die Bestimmung des Hautalters erfolgt insbesondere in folgenden Schritten:

a) Optoelektronische Erfassung eines Hautareals mit einer digitalen Kameraeinheit (DKE),

- wobei das zu erfassende Hautareal zusätzlich mit einer Lichtquelle (LQ) beleuchtet wird,
- wobei die digitale Kameraeinheit eine Kreuzpolarisationseinheit (KPE) aufweist,

b) Speicherung des Bildes in einem Zwischenspeicher (MEM),

c) Bildverarbeitung mittels mikroprozessorgesteuerter Bildverarbeitungseinheit (mBVE),

- wobei die mBVE auf das im Zwischenspeicher abgelegte Bild zugreift,
- in einem ersten Schritt den B-Kanal des RGB-Bildes isoliert und ein Grauwertbild errechnet und
- in einem zweiten Schritt den mittleren Grauwert aus dem Grauwertbild ermittelt und diesen als Parameter "Farbe" zwischenspeichert,
- in einem dritten Schritt oder zum zweiten Schritt parallelen Arbeitsprozess die Standardabweichung aus dem Histogramm der Grauwerte errechnet und diese als Parameter "Ebenheit" zwischenspeichert,

d) Berechnung des Hautalters mit der Farb-Altersabhängigkeitskorellations-Formel (FAF) anhand der Farbe, und/oder

e) Berechnung des Hautalters mit der Ebenheits-Altersabhängigkeits-Formel (EAF) anhand der Ebenheit und

f) Ausgabe und/oder Visualisierung des Hautalterswertes oder Nutzung des Hautalterswertes zur Produktempfehlung.

Vorzugsweise erfolgt die optoelektronische Erfassung als fotografische Aufnahme. Vorzugsweise erfolgt die Beleuchtung gerichtet.

Die im Verfahren eingesetzten Formeln zur Farb-Altersabhängigkeitskorrelation und Ebenheits-Altersabhängigkeitskorrelation werden experimentell bestimmt. Dazu werden eine bei einer genügend großen und altersmäßig breit verteilten Probandenzahl, die Werte für Farbe und Ebenheit nach dem oben genannten Verfahrensschritten a) bis c) bestimmt und grafisch oder rechnerisch ‚korreliert'. Das Ergebnis ist eine Mathematische Funktion, die FAF bzw. EAF. Mit Hilfe der FAF und EAF lassen sich im Verfahrensschritt d) und e) aus der gemessenen Farbe und Ebenheit das Hautalter berechnen.

Die Bestimmung (Berechnung) des auszugebenden Alters erfolgt vorzugsweise durch Mittelung der Ergebnisse aus Verfahrensschritten d) und e).

Die verwendeten Korrelationsverfahren basieren auf Regressionsanalysen wie sie z.B. im "Taschenbuch der Mathematik" von Ilja N. Bronstein, K. A. Semendjajew beschrieben werden und berücksichtigen im geeigneten Maße die biologische gegebene Streuung des/der Parameter.

Im letzten Schritt des Verfahrens, Schritt f), wird dem Probanden (am POS dem Verbraucher oder Konsument) das Messergebnis, also das errechnete Hautalter oder eine Produktempfehlung bekanntgegeben.

Die Bekanntgabe das Hautalters kann erfindungsgemäß vorteilhaft als Sprachausgabe oder visualisiert erfolgen. Besonders vorteilhaft ist eine visuelle Ausgabe auf einem Display oder einem angefertigtem Ausdruck.

Untersuchungen haben gezeigt, dass sich mit dem Alter die Farbe als auch der Evenskintone (Gleichmäßigkeit der Farbverteilung) ändern. Im Alter wird die Haut dunkler und ungleichmäßiger. Eine dunkle und gleichmäßige Haut weist dagegen auf den Bräunungsstatus bzw. auf die ethnische Zugehörigkeit hin. Ein durch Sommersprossen oder Altersflecken verursachter "unebener Hautton" ist allein nicht Aussagekräftig genug für eine Altersbestimmung, da bei einem heller Grundton bzw. Hauttyp ein stärkerer Kontrast vorhanden ist und daher der uneven skin tone überinterpretiert würde, was zu einem zu hohen Alter führt.

Um durch solche Einflüsse verursachte Fehler zu umgehen, ist es von Vorteil zur Bestimmung des auszugebenden Hautalters sowohl die Farbe und Ebenheit bzw. das sich aus Farbe und Ebenheit errechnete Alter heranzuziehen.

Die einfachste Möglichkeit ist hierbei die Anwendung einer linearen Korrelation. Besonders vorteilhaft ist es jedoch, anstelle der linearen Korrelation ein mathematisches oder komplexes Modell anzuwenden, in dem bei starker Pigmentierung die Ebenheit (Farbverteilung) stärker gewichtet wird als bei heller Hautfarbe. Eine hervorragende Methode ist auch der Einsatz eines neuronalen Netzwerkes, welches mit zunehmender Probandenzahl genauer wird.

Im Sinne des Verfahrens ist auch, der Ausgabe von Produktempfehlungen eine Abfrage von Informationen voranzustellen, so dass diese Informationen ebenfalls in die Produktauswahl einbezogen werden können. Besonders vorteilhaft ist es das wahren Alters und/oder die ethnische Zugehörigkeit und/oder Sonnenbelastung/-bräunung und/oder persönliche Einschätzung des Hautzustandes abzufragen.

Insbesondere die eine persönliche Einschätzung des Hautzustandes, wie z. B. trockene Haut oder normale Haut oder fettige Haut oder Akne, können wichtige Hinweise sein, um die Anzahl der vorzuschlagenden Produkte einzugrenzen.

Weitere Anhaltspunkte für eine richtige Produktauswahl werden erhalten, wenn zusätzlich zu den Parametern Farbe und Ebenheit die Hautfeuchte und/oder der Hautglanz bestimmt wird. Vorteilhaft ist es zudem zwei unterschiedliche Hautbereiche zu analysieren, um weitere Informationen über die Bräunung der Haut oder Ethnie zu erhalten. Dazu könnte zum Beispiel und das ist im Sinne der Erfindung besonders vorteilhaft, die grundlegende Analyse für ein Hautareal auf dem Handrücken erfolgen. Als Zusatzanalyse würde gleichzeitig oder anschließend ein Hautareal der Handinnenfläche analysiert. Aus der Differenz der Helligkeit lässt sich ableiten, ob der Proband durch Sonnenexposition gebräunt ist oder einem dunklen Phototyp (z.B. IV, V oder VI) angehört.

Anhand der unterschiedlichen Bräunung von Handrücken und Handfläche lässt sich feststellen, in wie weit die Bräunung künstlich durch Anwendung eines Solariums entstanden ist und nicht durch ethnische Einflüsse. Bei Bräunung im Solarium ist der Bräunungsunterschied zwischen Handfläche und Handrücken geringer als bei Personen dunkler Hautfarbe.

Das Verfahren kann unter Anwendung einzelner Vorrichtungen ausgeführt werden oder mittels einer Vorrichtung in denen alle für die Ausführung der Verfahrensschritte nötigen Untereinheiten integriert (zusammengefasst) sind.

Eine solche, integrierte Vorrichtung, ist als Altersscanner (Agescanner) zu bezeichnen und könnte z. B. am Point of Sale, also dem Verkaufspunkt, direkt vom Kunden genutzt werden um eine Hilfestellung bei der Produktauswahl zu erhalten.

Die Aufnahmen werden mittels einer hochauflösenden Digitalkamera die vorteilhafterweise über eine Software direkt angesprochen wird erfasst. Vorteilhafterweise wäre eine Speicherung des Bildes als nativer Datensatz im RAW-Datenformat vorstellbar, denkbar ist aber auch der Einsatz eines anderen Dateiformats mit verlustfreier oder geringer Kompressionsrate.

Um Umgebungseinflüsse auszuschließen, ist eine Beleuchtung mit kreuzpolarisiertem Licht vorteilhaft. Geeignete Lichtquellen sind zum Beispiel Diodenmatrixleuchten oder Elektronenblitze (Gasentladungsblitzgeräte), insbesondere im Zusammenspiel mit Hilfsmitteln wie Lichtwannen und Diffusoren.

Die Visualisierung des Hautalters oder der Produktempfehlung kann über eine Anzeigeeinheit oder einen Ausdruck erfolgen. Zur Anzeige eignen sich z. B. LCD-Matrixdisplays, 7-SegmentAnzeigen, Alphanumerische Displays, Dot-Matrix-Displays, TFT-Displays, FTN-Displays, Röhrenmonitore. Bevorzugt wegen ihres geringen Energieverbrauchs und ansprechender Darstellung sind TFT-Displays. Besonders bevorzugt weisen die Displays eine Touch-Screen-Funktionalität auf, mit denen zusätzlich zur reinen Ausgabe des Messergebnisses oder der Produktempfehlung eine Interaktion mit dem Verbraucher möglich ist.

Vorteilhafterweise werden jeweils z. B. drei Produkte auf der Visualisierungseinheit angezeigt oder auf einen Empfehlungszettel mit einem geeigneten technischen Verfahren gedruckt oder eine sofortige Auslieferung des Produkt mittels Automat ermöglicht. Neben den Produktangaben können in einer vorteilhaften Ausführung auch noch weitere Produktinformationen gegeben werden und/oder zusätzlich Kommunikationsabfragen wie z. B. e-Mailadresse des Nutzers abgefragt werden. Möglich ist auch eine drahtlose Übermittlung (z. B. IR, Bluetooth, SMS) des Ergebnisses zu einem Mobiltelefon des Kunden.

Im Folgenden wird das Verfahren und eine für die Durchführung des Verfahrens geeignete Vorrichtung anhand eines Aufführungsbeispiels beschrieben. Die Erfindung soll jedoch nicht auf das Ausführungsbeispiel eingeschränkt sein, welches nur zur visuellen Unterstützung der Beschreibung dient.

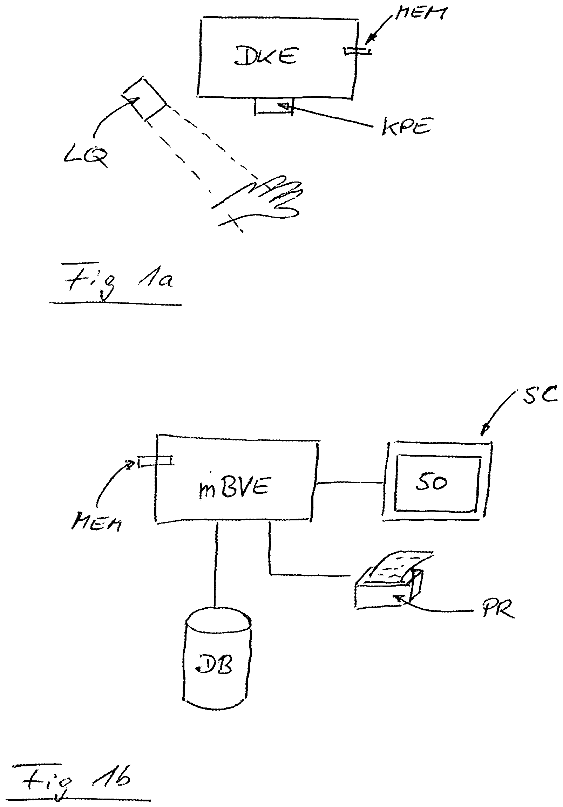

Figur 1a zeigt schematisch einen Aufbau der geeignet ist das Verfahren auszuführen. Mit der digitalen Kameraeinheit (DKE) wird eine fotografische Aufnahme eines zusätzlich mit einer Lichtquelle (LQ) beleuchteten Hauareals, hier des Handrückens, erstellt. Die Aufnahme wird auf einem Speicherchip, dem Zwischenspeicher (MEM) gespeichert. Die DKE ist mit einer Kreuzpolarisationseinheit ausgerüstet (KPE), durch deren

Einsatz die Oberflächenstruktur der Haut auf optischem Wege weites gehend ausgeblendet wird, so dass Hautunebenheiten und -farbe prägnanter zu Tage kommt.

Figur 1b zeigt schematisch eine Vorrichtung zur Bildanalyse mit mikroprozessorgesteuerter Bildverarbeitungseinheit (mBVE). Die auf dem Zwischenspeicher (MEM) gespeicherte Aufnahme, wird dazu so mit der mBVE verbunden, dass die nMBV auf das gespeicherte Bild zugreifen kann.

Die mBVE an im einfachsten Fall ein normaler Computer sein, auf dem mittels einer Bildanalysesoftware die Schritte c) bis f) des Verfahrens ausgeführt werden können.

Die Algorithmen zur Bestimmung des Parameters Farbe und des Parameters Ebenheit sind bereits im Stand der Technik bekannt da sie statistisch/mathematischen Grundwissen entspringen wie sie z. B. im Taschenbuch der Mathematik" von Ilja N. Bronstein, K. A. Semendjajew im Bereich der Regressionsanalyse nachgelesen werden können. Anschließend werden mit Hilfe der Farb-Altersabhängigkeitskorellations-Formel (FAF) anhand der Farbe und der Ebenheits-Altersabhängigkeits-Formel (EAF) anhand der Ebenheit das Hautalter des fotografierten Hautareals bestimmt. Das berechnete Hautalter wird auf der Anzeigeeinheit (SC) ausgegeben. In einer aufwendigeren Variante wird anhand des errechneten Hautalters unter Nutzung einer Datenbank (DB), die Informationen über Produkte in Verbindung mit den für die Produkte geeigneten Hautaltersbereichen enthält, die Produkte herausgesucht, die für das gemessene Hautalter in Frage kommen. Das Ergebnis der Datenbankabfrage kann dem Verbraucher auf einer Anzeigeeinheit oder als Ausdruck (PR) visualisiert werden.

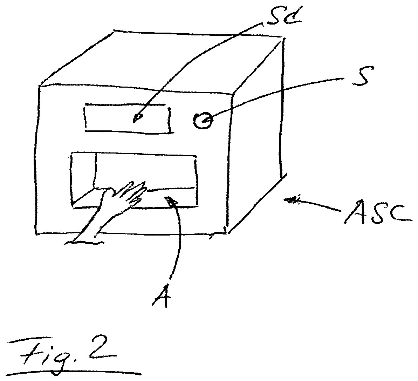

Figur 2 zeigt beispielhaft ein integriertes Gerät (Agescanner) (ASC), der im Prinzip die in Figur 1 dargestellten Komponenten aufweist. Der Verbraucher legt zur Altersbestimmung seine Hand auf die Auflagefläche (A). Zum Start der fotografischen Aufnahme eignen sich verschiedene Szenarien. Die einfachste Möglichkeit die Aufnahme zu starten ist, der Verbraucher startet die Aufnahme durch Betätigung eines Startschalters (S). Eleganter und besonders vorteilhaft ist es jedoch, wenn die Aufnahme selbsttätig gestartet wird, dazu wird nicht nur ein Einzelbild aufgenommen, sondern die Handoberfläche gefilmt, sobald die Auflagefläche berührt oder belastet wird. Auch die Detektion mittels einer Lichtschranke oder Wärmesensor ist möglich. Aus der Sequenz der Einzelbilder des Films wird ein Bild für die Weiterverarbeitung herangezogen. Vorteilhaft ist es mehr als ein Bild zur separaten Auswertung heranzuziehen, da eine Mittelung über mehrere Farbwerte und Ebenheitswerte zur gesicherteren Altersbestimmung führt.

Das errechnete Hautalter oder die mittels Datenbank ermittelte Produktempfehlung wird dem Verbraucher auf einem Anzeigeeinheit (SC) visualisiert.

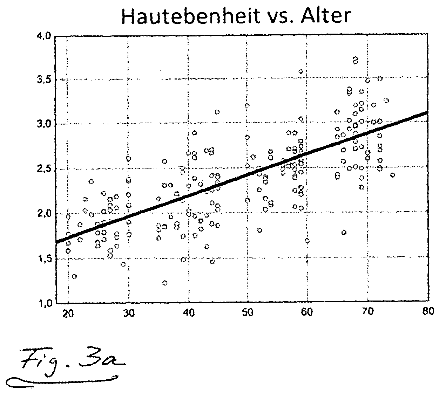

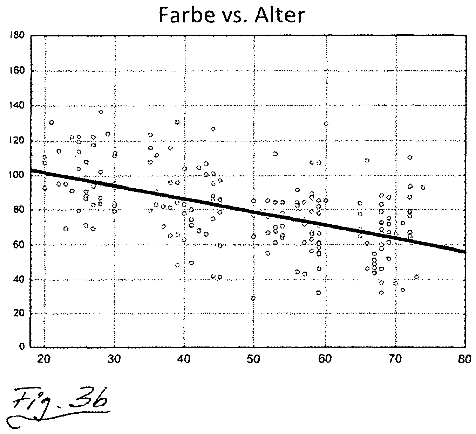

Figur 3 zeigt beispielhaft die Auswertung eines vermessenen Kollektives, insbesondere die Abhängigkeit der Farbe vom Alter (Figur 3b) und der Ebenheit (Farbverteilung) in Abhängigkeit des Alters (Figur 3a), wobei jeweils auf der Abszisse das Alter und auf der Ordinate die Ebenheit (3a) bzw. die Farbe (3b) aufgetragen ist.

Eine lineare Regression führt zu folgender Farb-Altersabhängigkeitskorellations-Formel (FAF)

$$\text{Alter} = 116{,}65 - 0{,}76 * \text{Farbe}$$

und zu folgender Ebenheits-Altersabhängigkeits-Formel (EAF)

$$\text{Alter} = 1{,}25 + 0{,}02 * \text{Ebenheit}$$

**Patentansprüche**

1. Verfahren zur Bestimmung des Hautalters aufweisend zumindest folgende Schritte:

   a) Optoelektronische Erfassung eines Hautareals mit einer digitalen Kameraeinheit (DKE),

   - wobei das zu erfassende Hautareal zusätzlich mit einer Lichtquelle (LQ) beleuchtet wird,
   - wobei die digitale Kameraeinheit (DKE) eine Kreuzpolarisationseinheit (KPE) aufweist,

   b) Speicherung des Bildes in einem Zwischenspeicher (MEM),
   c) Bildverarbeitung mittels mikroprozessorgesteuerter Bildverarbeitungseinheit (mBVE),

   - wobei die mikroprozessorgesteuerter Bildverarbeitungseinheit auf das im Zwischenspeicher abgelegte Bild zugreift,
   - in einem ersten Schritt den B-Kanal des RGB-Bildes isoliert und so ein Grauwertbild errechnet und
   - in einem zweiten Schritt den mittleren Grauwert aus dem Grauwertbild ermittelt und diesen als einen ersten Parameter zwischenspeichert,
   - in einem dritten Schritt oder zum zweiten Schritt parallelen Arbeitsprozess die Standardabweichung aus dem Histogramm der Grauwerte errechnet und diese als einen zweiten Parameter zwischenspeichert,

   d) Berechnung des Hautalters mit einer Farb-Altersabhängigkeitskorellations-Formel (FAF) anhand des ersten Parameters,
   e) Berechnung des Hautalters mit einer Ebenheits-Altersabhängigkeits-Formel (EAF) anhand des zweiten Parameters und
   f) Ausgabe eines Hautalterswertes oder Nutzung des Hautalterswertes (HA) zur Produktempfehlung.

2. Verfahren nach Anspruch 1, **dadurch gekennzeichnet, dass** die optoelektronische Erfassung mit der digitalen Kameraeinheit als Fotografische Aufnahme erfolgt.

3. Verfahren nach mindestens einem der vorhergehenden Ansprüche, **dadurch gekennzeichnet, dass** die Beleuchtung mit der Lichtquelle des zu fotografierenden Hautareals gerichtet erfolgt.

4. Verfahren nach mindestens einem der vorhergehenden Ansprüche, **dadurch gekennzeichnet, dass** zur Auswertung mehr als eine fotografische Aufnahme mit der digitalen Kameraeinheit herangezogen wird.

5. Verfahren nach mindestens einem der vorhergehenden Ansprüche, **dadurch gekennzeichnet, dass** die Ausgabe des gemessenen Hautalters visualisiert und/oder als Sprachausgabe erfolgt.

6. Verfahren nach mindestens einem der vorhergehenden Ansprüche, **dadurch gekennzeichnet, dass** anhand des gemessenen Alters (HA) auf dieses Alter abgestimmte Produktvorschläge gemacht werden, wobei aus einer Reiche von Produkte ausgewählt wird, die in einer Datenbank gespeichert sind.

**7.** Verfahren nach mindestens einem der vorhergehenden Ansprüche, **dadurch gekennzeichnet, dass** das wahre Alter in die Betrachtung der Produktauswahl einbezogen wird und zur Produktauswahl ein Zwischenwert zwischen gemessenen Alter und wahrem Alter verwendet wird, insbesondere der Mittelwert von Wahrem zu gemessenem Alter verwendet wird.

**8.** Verfahren nach mindestens einem der vorhergehenden Ansprüche, **dadurch gekennzeichnet, dass** zur Berechnung des Hautalters Farbe und/oder Ebenheit von zwei unterschiedlichen Körperregionen verwendet werden.

**9.** Verfahren nach mindestens einem der vorhergehenden Ansprüche, **dadurch gekennzeichnet, dass** zur Berechnung des Hautalters Farbe und/oder Ebenheit von Handrücken und Handinnenfläche verwendet werden.

**10.** Verfahren nach mindestens einem der vorhergehenden Ansprüche, **dadurch gekennzeichnet, dass** die zur Berechnung der Farb-Altersabhängigkeitskorellations-Formel (FAF) und Ebenheits-Altersabhängigkeits-Formel (EAF) eingesetzte Formel aus einem Kollektiv von Messwerten der Farbe in Abhängigkeit vom wahren Alter für die Farb-Altersabhängigkeitskorellations-Formel und Ebenheit in Abhängigkeit vom Alter für die Ebenheits-Altersabhängigkeits-Formel bestimmt wurde.

**11.** Vorrichtung zur Bestimmung des Hautalters nach Verfahren der Ansprüche 1 bis 9 aufweisend:

Digitale Kameraeinheit (DKE) mit Kreuzpolarisationseinheit (KPE) zur optoelektronischen Erfassung eines Hautareals,
Zwischenspeicher zur Zwischenspeicherung mindestens einer fotografischen Aufnahme und Mikroprozessorgesteuerte Bildverarbeitungseinheit (mBVE) zur Auswertung der fotografischen Aufnahme, wobei die mikroprozessorgesteuerte Bildverarbeitungseinheit derart eingerichtet ist, dass sie

- auf das im Zwischenspeicher abgelegte Bild zugreift und
- in einem ersten Schritt den B-Kanal des RGB-Bildes isoliert und so ein Grauwertbild ermittelt,
- in einem zweiten Schritt den mittleren Grauwert aus dem Grauwertbild ermittelt und diesen als einen ersten Parameter zwischenspeichert,
- in einem dritten Schritt oder zum zweiten Schritt parallelen Arbeitsprozess die Standardabweichung aus dem Histogramm der Grauwerte errechnet und diese als einen zweiten Parameter zwischenspeichert, und
- das Hautalter mit der Farb-Altersabhängigkeitskorellations-Formel (FAF) anhand des ersten Parameters berechnet und das Hautalter mit der Ebenheits-Altersabhängigkeits-Formel (EAF) anhand des zweiten Parameters berechnet.

**12.** Vorrichtung nach Anspruch 11, **dadurch gekennzeichnet, dass** die optoelektronische Erfassung als fotografischen Aufnahme erfolgt.

**13.** Vorrichtung nach mindestens einem der Ansprüche 11 bis 12, **dadurch gekennzeichnet, dass** sie ein Display oder eine Anzeige aufweist.

**14.** Vorrichtung nach mindestens einem der Ansprüche 11 bis 13, **dadurch gekennzeichnet, dass** sie eine Sprachausgabeeinheit aufweist

**15.** Vorrichtung nach mindestens einem der Ansprüche 11 bis 14, **dadurch gekennzeichnet, dass** sie eine Lichtquelle aufweist.

**16.** Vorrichtung nach mindestens einem der Ansprüche 11 bis 15, **dadurch gekennzeichnet, dass** sie eine Datenbank für Produktinformationen ausweist, wobei zu den Produkten ein Altersbezug und/oder eine Angabe für welches Hautalter das Produkt geeignet ist, gespeichert ist.

**Claims**

**1.** A method for determining the age of the skin, comprising at least the following steps:

a) optoelectronic capture of an area of skin using a digital camera unit (DKE),
wherein the area of skin to be captured is additionally illuminated by a light source (LQ),
wherein the digital camera unit (DKE) has a cross-polarization unit (KPE),
b) storing the image in a buffer (MEM),
c) processing the image by means of a microprocessor-controlled image processing unit (mBVE),

- wherein the microprocessor-controlled image processing unit accesses the image stored in the buffer,
- isolates the B channel of the RGB image and calculates a grayscale value image in a first step and
- establishes the mean grayscale value from the grayscale value image and buffers it as a first parameter in a second step,

- calculates the standard deviation from the grayscale value histogram and buffers it as a second parameter in a third step or in a work process parallel to the second step,

d) calculating the age of the skin using the color/age-dependence correlation formula (FAF) based on the first parameter,
e) calculating the age of the skin using the evenness/age-dependence formula (EAF) based on the second parameter, and
f) outputting a value for the age of the skin or using the value for the age of the skin (HA) in order to recommend a product.

2. The method as claimed in claim 1, **characterized in that** the optoelectronic capture using the digital camera unit is brought about as a photographic recording.

3. The method as claimed in at least one of the preceding claims, **characterized in that** the illumination using the light source of the area of skin to be photographed is brought about in a directed fashion.

4. The method as claimed in at least one of the preceding claims, **characterized in that** more than one photographic recording using the digital camera unit is used for evaluation purposes.

5. The method as claimed in at least one of the preceding claims, **characterized in that** the output of the measured age of the skin is visualized and/or is brought about as speech output.

6. The method as claimed in at least one of the preceding claims, **characterized in that** the measured age (HA) is used to make product suggestions matched to this age, wherein a selection is made from a range of products stored in a database.

7. The method as claimed in at least one of the preceding claims, **characterized in that** the true age is included when considering the product selection and an intermediate value between measured age and true age is used for product selection, more particularly the mean value between true and measured age.

8. The method as claimed in at least one of the preceding claims, **characterized in that** color and/or evenness of two different body regions are used to calculate the age of the skin.

9. The method as claimed in at least one of the preceding claims, **characterized in that** color and/or evenness of the back of the hand and the palm of the hand are used to calculate the age of the skin.

10. The method as claimed in at least one of the preceding claims, **characterized in that** the formula used to calculate the color/age-dependence correlation formula (FAF) and the evenness/age-dependence formula (EAF) was determined from a collective of measurement values of the color depending on the true age for the color/age-dependence correlation formula and the evenness depending on the age for the evenness/age-dependence formula.

11. A device for determining the age of the skin as claimed in the method of claims 1 to 9, comprising:

a digital camera unit (DKE) with a cross-polarization unit (KPE) for optoelectronic capture of an area of skin,
a buffer for buffering at least one photographic recording, and a microprocessor-controlled image processing unit (mBVE) for evaluating the photographic recording, wherein the microprocessor-controlled image processing unit is configured to

- access the image stored in the buffer,
- isolate the B channel of the RGB image and thus calculate a grayscale value image in a first step and
- establish the mean grayscale value from the grayscale value image and buffer it as a first parameter in a second step,
- calculate the standard deviation from the grayscale value histogram and buffer it as a second parameter in a third step or in a work process parallel to the second step, and
- calculates the age of the skin using the color/age-dependence correlation formula (FAF) based on the first parameter, and calculates the age of the skin using the evenness/age-dependence formula (EAF) based on the second parameter.

12. The device as claimed in claim 11, **characterized in that** the optoelectronic capture is brought about as a photographic recording.

13. The device as claimed in at least one of claims 11 to 12, **characterized in that** it has a display or an indicator.

14. The device as claimed in at least one of claims 11 to 13, **characterized in that** it has a speech output unit.

15. The device as claimed in at least one of claims 11 to 14, **characterized in that** it has a light source.

16. The device as claimed in at least one of claims 11 to 15, **characterized in that** it has a database for product information, wherein an age reference and/or a specification relating to the age of the skin for which the product is suitable are stored with the products.

**Revendications**

1. Procédé destiné à la détermination du vieillissement de la peau présentant tout au moins les étapes suivantes :

   a) détection optoélectronique d'une zone de la peau au moyen d'une unité de caméra numérique (DKE) ;

      - où la zone de la peau à détecter est en outre éclairée par une source de lumière (LQ);
      - où l'unité de caméra numérique (DKE) présente une unité de polarisation en croisement (KPE);

   b) enregistrement de l'image dans une unité de mémoire tampon (MEM) ;
   c) traitement de l'image au moyen d'une unité de traitement des images contrôlée par un microprocesseur (mBVE) ;

      - selon lequel l'unité de traitement des images contrôlée par un microprocesseur accède à l'image archivée dans l'unité de mémoire tampon ;
      - selon lequel le canal B de l'image RGB est isolé, dans une première étape, et selon lequel une image est ainsi calculée en niveau de gris ;
      - selon lequel la valeur de niveau de gris moyen est déterminée à partir de l'image en niveau de gris, dans une deuxième étape, et celle-ci étant placée en mémoire tampon en tant que premier paramètre ; et
      - selon lequel la déviation standard est calculée à partir de l'histogramme des valeurs de niveau de gris, dans une troisième étape ou bien dans un processus de travail réalisé parallèlement à la deuxième étape, et celle-ci étant placée en mémoire tampon en tant que deuxième paramètre ;

   d) détermination du vieillissement de la peau au moyen d'une formule de corrélation élaborée en fonction du vieillissement et de la coloration de la peau (FAF), à l'aide du premier paramètre ;
   e) détermination du vieillissement de la peau au moyen d'une formule de corrélation élaborée en fonction du vieillissement et de la densification de la peau (EAF), à l'aide du deuxième paramètre ; et
   f) émission d'une valeur de vieillissement de la peau ou utilisation de la valeur de vieillissement de la peau (HA) à des fins de recommandation de produits.

2. Procédé selon la revendication 1, **caractérisé en ce que** la détection optoélectronique est réalisée comme une prise de vue photographique, au moyen de l'unité de caméra numérique.

3. Procédé selon au moins l'une des revendications précédentes, **caractérisé en ce que** l'éclairage est réalisé avec la source de lumière de la zone de la peau devant être photographiée.

4. Procédé selon au moins l'une des revendications précédentes, **caractérisé en ce que** plus d'une prise de vue photographique est mise en oeuvre au moyen de l'unité de caméra numérique à des fins d'évaluation.

5. Procédé selon au moins l'une des revendications précédentes, **caractérisé en ce que** la valeur émise du vieillissement mesuré de la peau est visualisée et/ou est réalisée en tant que signal vocal.

6. Procédé selon au moins l'une des revendications précédentes, **caractérisé en ce que**, à partir du vieillissement mesuré (HA), des propositions de produits adaptés à ce vieillissement sont effectuées, une sélection étant réalisée à partir d'une gamme de produits qui sont enregistrés dans une base de données.

7. Procédé selon au moins l'une des revendications précédentes, **caractérisé en ce que** le véritable vieillissement est pris en compte en vue de déterminer la sélection de produits et une valeur intermédiaire située entre le vieillissement mesuré et le vieillissement véritable est employée à des fins de sélection des produits, en particulier la valeur moyenne est employée en partant du vieillissement véritable vers le vieillissement mesuré.

8. Procédé selon au moins l'une des revendications précédentes, **caractérisé en ce que** la coloration et/ou la densification de l'épiderme sont employées à partir de deux zones différentes de la peau en vue de la détermination du vieillissement de la peau.

9. Procédé selon au moins l'une des revendications précédentes, **caractérisé en ce que** la coloration et/ou la densification de l'épiderme sont employées à partir du dos de la main et de la paume de la main en vue de la détermination du vieillissement de la peau.

**10.** Procédé selon au moins l'une des revendications précédentes, **caractérisé en ce que** la formule utilisée, destinée à la détermination de la formule de la corrélation élaborée en fonction du vieillissement et de la coloration de la peau (FAF), d'une part, et de la formule de la corrélation élaborée en fonction du vieillissement et de la densification de la peau (EAF), d'autre part, a été déterminée à partir d'un collectif de valeurs de mesure de la coloration de l'épiderme en fonction du vieillissement véritable pour la formule de la corrélation élaborée en fonction du vieillissement et de la coloration de la peau, d'une part, et de la densification de l'épiderme en fonction du vieillissement pour la formule de la corrélation élaborée en fonction du vieillissement et de la densification de la peau, d'autre part.

**11.** Dispositif destiné à la détermination du vieillissement de la peau selon le procédé des revendications 1 à 9 présentant :

a) une unité de caméra numérique (DKE) avec une unité de polarisation en croisement (KPE) en vue de la détection optoélectronique d'une zone de la peau ;

b) une unité de mémoire tampon en vue de la mise en mémoire tampon d'au moins une prise de vue photographique et une unité de traitement des images contrôlée par un microprocesseur (mBVE) en vue de l'évaluation de la prise de vue photographique, l'unité de traitement des images contrôlée par un microprocesseur étant configurée de telle manière qu'elle:

- accède à l'image archivée dans l'unité de mémoire tampon et isole le canal B de l'image RGB, dans une première étape, et calcule ainsi une image en niveau de gris ;

- détermine la valeur de niveau de gris moyen à partir de l'image en niveau de gris, dans une deuxième étape, et place celle-ci en mémoire tampon en tant que premier paramètre ;

- calcule la déviation standard à partir de l'histogramme des valeurs de niveau de gris, dans une troisième étape ou bien dans un processus de travail réalisé parallèlement à la deuxième étape, et place celle-ci en mémoire tampon en tant que deuxième paramètre ; et

- calcule le vieillissement de la peau au moyen de la formule de corrélation élaborée en fonction du vieillissement et de la coloration de la peau (FAF), à l'aide du premier paramètre, et calcule également le vieillissement de la peau au moyen de la formule de corrélation élaborée en fonction du vieillissement et de la densification de la peau (EAF), à l'aide du deuxième paramètre.

**12.** Dispositif selon la revendication 11, **caractérisé en ce que** la détection optoélectronique est réalisée en tant que prise de vue photographique.

**13.** Dispositif selon au moins l'une des revendications 11 à 12, **caractérisé en ce que** ledit dispositif présente un écran ou un affichage.

**14.** Dispositif selon au moins l'une des revendications 11 à 13, **caractérisé en ce que** ledit dispositif présente une unité de signal vocal.

**15.** Dispositif selon au moins l'une des revendications 11 à 14, **caractérisé en ce que** ledit dispositif présente une source de lumière.

**16.** Dispositif selon au moins l'une des revendications 11 à 15, **caractérisé en ce que** ledit dispositif comprend une base de données pour des informations de produit, où sont enregistrées, par rapport aux produits, une référence au vieillissement et/ou une indication spécifiant pour quel vieillissement de la peau le produit est adéquat.

MEM

DKE

KPE

LQ

Fig 1a

SC

MEM

mBVE

SO

DB

PR

Fig 1b

Fig. 2

Hautebenheit vs. Alter

Fig. 3a

## Farbe vs. Alter

Fig. 36

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2008003146 A2